# EUROPEAN PATENT APPLICATION

(11) **EP 4 666 950 A1**
(43) Date of publication of application: **24.12.2025**
(21) Application number: 24755187.2
(22) Date of filing: 14.02.2024
(51) Int. Cl.: A61B 6/03

(54) **PLAIN CT IMAGING AID SYSTEM**

(30) Priority: 14.02.2023 JP 2023020443
(71) Applicant: Juntendo Educational Foundation, Tokyo 113-8421 (JP)
(72) Inventor: KAWAI, Satoru, Tokyo 113-8421 (JP); SUZUKI, Michimasa, Tokyo 113-8421 (JP); KYOGOKU, Shinsuke, Tokyo 113-8421 (JP); KIKUCHI, Nao, Tokyo 113-8421 (JP); HOJO, Noboru, Tokyo 113-8421 (JP); ABE, Kazuya, Tokyo 113-8421 (JP)
(74) Representative: Plougmann Vingtoft a/s
(86) International application number: PCT/JP2024/004943
(87) International publication number: WO 2024/172053

(57) **Abstract**

An object of the present invention is to provide an aid system capable of accurate diagnosis in a non-contrast CT imaging diagnosis even when a difference in CT values is minute as in cases such as immediately after onset. The present invention relates to a non-contrast CT imaging diagnostic aid system including: (1) means for acquiring a non-contrast CT image; and (2) means of applying a non-linear transformation to a CT value of each pixel in the CT image to reconstruct the CT image.

## Description

### Technical Field

The present invention relates to a non-contrast CT imaging diagnostic aid system and a non-contrast CT imaging diagnostic aid method.

### Background Art

The MRI, which is a standard for diagnosis of cerebral infarction and the like, is in high demand for examination and is difficult to make an appointment. In addition, since the examination takes time and laboratories are not always immediately available, it is often the case that even a disease in the acute stage cannot immediately be examined. In such cases, the non-contrast CT which requires no contrast agent may be performed first. However, it is unusual that definite diagnosis of acute-stage cerebral infarction is established only by the non-contrast CT, so that the diagnosis is confirmed as a result of the MRI. For example, the reason is that, in hyperacute cerebral infarction, immediate treatment significantly affects a patient quality of life (QOL).

For example, an early CT sign and a hyperdense MCA sign are as determinative factors for diagnosis in CT image findings of hyperacute cerebral infarction. However, the early CT sign immediately after onset is as small as 2 to 10 in terms of difference in CT values from normal sites, and on not a few occasions, even experienced doctors are confused in diagnosis.

### Summary of Invention

### Technical Problem

Accordingly, there is a demand for an aid system capable of accurate diagnosis in non-contrast CT imaging diagnosis even when a difference in CT values is minute as in cases such as immediately after onset.

### Solution to Problem

The inventors, who tested various types of image processing to enhance CT images, have found completely unexpectedly that a minute difference in CT values in cases such as immediately after onset is displayed as a more distinguishable difference by raising a CT value of each pixel in a CT image to a power and reconstructing the CT image based on the resultant raised CT value, so that it is possible to aid the non-contrast CT imaging diagnosis, and thus the present invention has been completed.

The present invention provides the following [1] to [14] :
[1] A non-contrast CT imaging diagnostic aid system including: (1) means for acquiring a non-contrast CT image; and (2) means of applying a non-linear transformation to a CT value of each pixel in the CT image to reconstruct the CT image.
[2] The non-contrast imaging diagnostic aid system according to [1], wherein the means of applying a non-linear transformation is means of raising a CT value of each pixel in a CT image to a power to reconstruct the CT image.
[3] The non-contrast CT imaging diagnostic aid system according to [1] or [2], further comprising means for displaying the CT image which was reconstructed in a color map.
[4] The non-contrast CT imaging diagnostic aid system according to any one of [1] to [3], wherein when applying a non-linear transformation to the CT value, at least one of pre-processing for replacing a CT value smaller than or equal to 0 with a value of zero or a near-zero value, pre-processing for replacing a CT value which causes overflow as a result of non-linear transformation with a value which does not cause overflow, and pre-processing for evenly shifting all CT values to a range in which an overflow does not occur is performed.
[5] The non-contrast CT imaging diagnostic aid system according to any one of [1] to [4], wherein the non-contrast CT image is a non-contrast CT image of a patient selected from the group consisting of a non-contrast CT image of hyperacute cerebral infarction, a non-contrast CT image of subarachnoid hemorrhage, a non-contrast CT image of brain tumor, a non-contrast CT image of brain metastasis, a non-contrast CT image of liver metastasis of cancer, and a non-contrast CT image of detailed examination of a vascular wall of a patient with arterial dissection.
[6] The non-contrast CT imaging diagnostic aid system according to any one of [1] to [4], wherein the non-contrast CT image is a non-contrast CT image of a patient selected from the group consisting of a non-contrast CT image of hyperacute cerebral infarction and a non-contrast CT image of brain tumor.
[7] The non-contrast CT imaging diagnostic aid system according to any one of [1] to [6], including: an artificial intelligence built through learning by using disease classification based on a non-contrast CT image as teaching data and using a CT image reconstructed by the means of reconstructing as training data; and means of predicting disease classification based on the non-contrast CT image acquired by the means for acquiring by reconstructing the non-contrast CT image by the means of reconstructing and inputting the reconstructed non-contrast CT image into the artificial intelligence.
[8] A non-contrast CT imaging diagnostic aid method, including: (1a) a step of acquiring a non-contrast CT image; and (2a) a step of applying a non-linear transformation to a CT value of each pixel in the CT image to reconstruct the CT image.
[9] The non-contrast imaging diagnostic aid method according to [8], wherein the step of applying a non-linear transformation is a step of raising a CT value of each pixel in the CT image to a power to reconstruct the CT image.
[10] The non-contrast CT imaging diagnostic aid method according to [8] or [9], further including means for displaying the CT image which was reconstructed in a color map.
[11] The non-contrast CT imaging diagnostic aid method according to any one of [8] to [10], wherein when applying a non-linear transformation to the CT value, at least one of pre-processing for replacing a CT value smaller than or equal to 0 with a value of zero or a near-zero value, pre-processing for replacing a CT value which causes overflow as a result of non-linear transformation with a value which does not cause overflow, and pre-processing for evenly shifting all CT values to a range in which an overflow does not occur is performed.
[12] The non-contrast CT imaging diagnostic aid method according to any one of [8] to [11], wherein the non-contrast CT image is a non-contrast CT image of a patient selected from the group consisting of a non-contrast CT image of hyperacute cerebral infarction, a non-contrast CT image of subarachnoid hemorrhage, a non-contrast CT image of brain tumor, a non-contrast CT image of brain metastasis, a non-contrast CT image of liver metastasis, and a non-contrast CT image of detailed examination of a vascular wall of a patient with arterial dissection.
[13] The non-contrast CT imaging diagnostic aid system according to any one of [8] to [11], wherein the non-contrast CT image is a non-contrast CT image of a patient selected from the group consisting of a non-contrast CT image of hyperacute cerebral infarction and a non-contrast CT image of brain tumor.
[14] The non-contrast CT imaging diagnostic aid method according to any one of [8] to [13], including: an artificial intelligence built through learning by using disease classification based on a non-contrast CT image as teaching data and using a CT image reconstructed by the step of reconstructing as training data; and a step of predicting disease classification based on the non-contrast CT image acquired by the step of acquiring by reconstructing the non-contrast CT image by the step of reconstructing and inputting the reconstructed non-contrast CT image to the artificial intelligence.

### Advantageous Effect of Invention

According to a non-contrast CT imaging diagnostic aid system and method of the present invention, a minute difference in CT values in a non-contrast CT image in hyperacute cerebral infarction, subarachnoid hemorrhage, brain tumor, brain metastasis of cancer, liver metastasis of cancer, detailed examination of a vascular wall of a patient with arterial dissection, and the like can be displayed as a more distinguishable difference, so that it is possible to find a patient with the disease early and deliver appropriate treatment.

### Brief Description of Drawings

Figure 1 illustrates CT values in an original CT image and CT values after raising to a power.
Figure 2 shows an original CT image, a CT image after raising the CT value to a power, and a CT image which is displayed in a color map of the CT image after raising to a power.
Figure 3 illustrates an example configuration of a non-contrast CT imaging diagnostic aid system.

### Description of Embodiments

The present invention relates to a non-contrast CT imaging diagnostic aid system and a non-contrast CT imaging diagnostic aid method.

A non-contrast computed tomography (CT) image refers to an image which is obtained without using any contrast agent and by detecting X-rays radiated and transmitted from various angles, collecting data, and subjecting the collected data to back projection by a computer. That is, it is an image which is projected, after X-rays are radiated, as an image corresponding to the degree of absorption of X-rays. Specifically, materials with high X-ray absorption coefficients (high density area) appear white in the image (bone, calcification, hematoma, metal, and the like), and materials with low absorption coefficients (low density area) appear black in the image (cerebrospinal fluid (brain ventricles), infarction, fat, and the like). The brightness or darkness of a color is relied on to diagnose the presence or absence of infarcted site, the size thereof, and the like.

The non-contrast CT imaging diagnostic aid system and the non-contrast CT imaging diagnostic aid method of the present invention are a system and a method for aiding diagnosis by enhancing the brightness or darkness of a color in CT images.

An aspect of the present invention is a non-contrast CT imaging diagnostic aid system, including: (1) means for acquiring a non-contrast CT image; and (2) means of applying a non-linear transformation to a CT value of each pixel in the CT image to reconstruct the CT image.

Another aspect of the present invention is a non-contrast CT imaging diagnostic aid method, including: (1a) a step of acquiring a non-contrast CT image; and (2a) a step of applying a non-linear transformation to a CT value of each pixel in the CT image to reconstruct the CT image.

The means and steps described above will be collectively described below.

Means (1) (step (1a)) is means (a method) of acquiring a non-contrast CT image.

The means (1) is means for acquiring a CT image by a typical CT scanner: a radiation source and a detector rotate around an examined subject, and the examined subject receives X-rays in all directions. The radiated X-rays, which pass through the examined subject, are partially absorbed by the subject and attenuated, and thereafter, reach an X-ray detecting device located on the opposite side of the radiation source and are then recorded. How much x-rays are absorbed in each direction is recorded, and thereafter, the image can be acquired by reconstructing it by a computer through Fourier transformation.

Means (2) (step (2a)) is means (a step) of applying a non-linear transformation to a CT value of each pixel in a CT image to reconstruct the CT image.

The unit of CT value is a Hounsfield unit (hereinafter abbreviated as "HU"). The CT value is represented as a relative value when the CT value is - 1000 HU for air and 0 HU for water. Negative numerical values (from 0 to -100) indicate fat.

The original CT value is transformed into a non-linear value through a non-linear transformation. The non-linear transformation may be a monotonically increasing function (which can be implemented by using a conversion table and the like in place of the function). In this way, the magnitude relationship between values before and after the transformation is maintained. Furthermore, as the original value is larger, it is possible to select a function which increases the extent of "transformed value - original value" (a function with which the first derivative is monotonically increasing).

Specific examples include a power function (power function is to calculate Xⁿ, with a certain numeral X: n may be an integer of greater than or equal to 2 or may be a real number greater than 1) and an exponential function (which calculates a^{X} with a certain numeral X: a is a real number greater than or equal to 1).

In the present invention, it is preferable to adopt means of raising a CT value of each pixel in a CT image to a power.

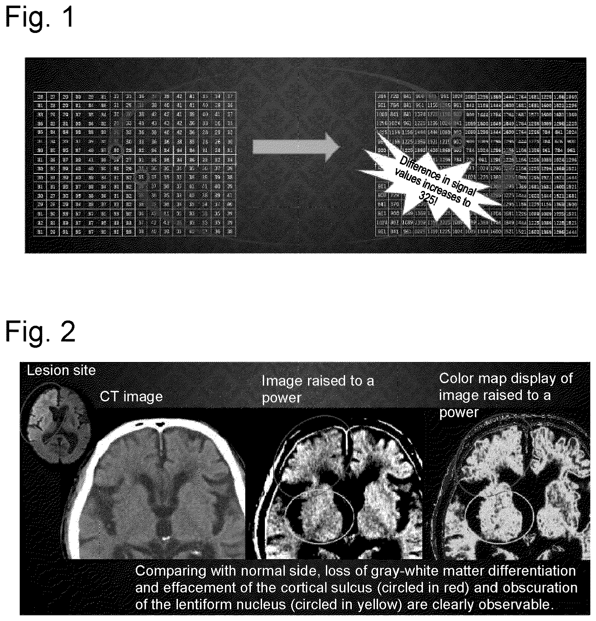

Figure 1 illustrates an example of raising a CT value of each pixel in a CT image to a power. For example, an original image CT value 30 is transformed into 900 and an original image CT value 35 is transformed into 1225. Here, the raising to a power is generally raising to the power of two, and may be raising to the power of three as necessary.

Many DICOM images, which are CT images, are 16-bit, which may cause overflow through raising to a power. Furthermore, by raising to a power negative CT values may be altered to positive numbers in some cases. That is, since the range of an unsigned 16-bit integer is from 0 to 65,535, an overflow may occur when the original CT value is greater than or equal to 256 in the case of raising to the power of two, and since the range of a signed 16-bit integer is from -32,768 to 32,767, an overflow may occur when the original CT value is greater than or equal to 182 in the case of raising to the power of two.

Accordingly, when applying a non-linear transformation to the CT value, it is preferable to perform at least one of pre-processing for replacing a CT value smaller than or equal to 0 with a value of zero or a near-zero value (which may be any value to the extent that it does not affect diagnosis in areas in which a CT value is not replaced, for example, it can be smaller than or equal to 1), pre-processing for replacing a CT value which causes overflow as a result of non-linear transformation with a value which does not cause overflow (which may be any value to the extent that it does not affect diagnosis in areas in which a Ct value is not replaced, for example, it can be a sufficiently large value without overflow or a value of zero or a near-zero value), and pre-processing for evenly shifting all CT values to a range in which an overflow does not occur (which can be accomplished by adding or subtracting a certain value).

Specifically, when raising the CT value to a power, as necessary, it is preferable to replace a CT value smaller than or equal to 0 with zero, replace a CT value which causes overflow as a result of raising to a power with zero, or to shift all CT values to a target range by addition or subtraction when the target will cause overflow as a result of raising to a power.

The CT image which was reconstructed by this means (step) is illustrated in Figure 2.

As illustrated in Figure 2, a minute difference in CT values can be displayed as a more distinguishable difference in the CT image which was reconstructed as compared with the original CT image.

The non-contrast CT imaging diagnostic aid system (method) of the present invention may further include means (step) of displaying the CT image which was reconstructed in a color map. Such a color map display may be implemented by using a function incorporated in a workstation such as VINCENT and ZIO station.

As illustrated in Figure 2, such a color map display makes it possible to further enhance the brightness or darkness of a color in the CT image for display, so that more appropriate diagnosis is possible.

In the system of the present invention, the non-contrast CT imaging diagnostic aid system may be made to include: an artificial intelligence built through learning by using disease classification based on a non-contrast CT image as teaching data and using a CT image reconstructed by the means of reconstructing as training data; and means of predicting disease classification based on the non-contrast CT image acquired by the means for acquiring by reconstructing the non-contrast CT image by the means of reconstructing, and inputting the reconstructed non-contrast CT image to the artificial intelligence.

The system of the present invention includes, for example, one or more processors and may be implemented by the processor performing a variety of required processes such as building-up through learning as described above, reconstruction of a non-contrast CT image, and prediction of disease classification.

Furthermore, in the method of the present invention, the non-contrast CT imaging diagnostic aid method may be made to include: an artificial intelligence built through learning by using disease classification based on a non-contrast CT image as teaching data and using a CT image reconstructed by the step of reconstructing as training data; and a step of predicting disease classification based on the non-contrast CT image acquired by the step of acquiring by reconstructing the non-contrast CT image by the step of reconstructing and inputting the reconstructed non-contrast CT image to the artificial intelligence.

A specific example of the non-contrast CT imaging diagnosis system of the present invention will be described with reference to a block diagram.

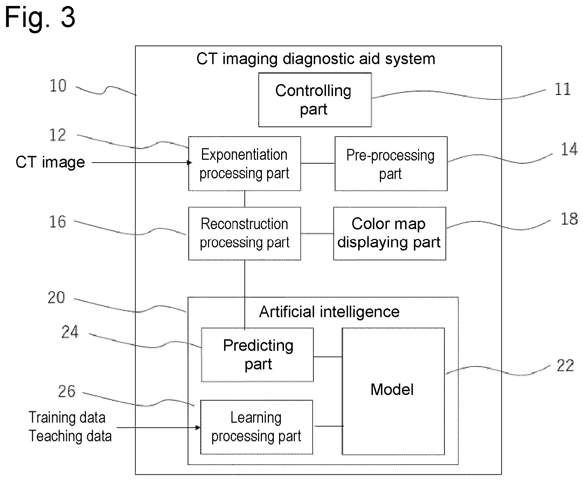

Figure 3 is a block diagram illustrating an example configuration of a non-contrast CT imaging diagnostic aid system 10.

The non-contrast CT imaging diagnostic aid system 10 can be implemented by using a computer such as a PC or a workstation. The computer includes a computing device, a computing device such as a central processing unit (CPU) and a graphics processing unit (GPU), a storage device such as a memory, and an input/output device such as a touch-panel display. The non-contrast CT imaging diagnostic aid system 10 is built up by controlling the computer by software.

The CT imaging diagnostic aid system 10 includes a controlling part 11, an exponentiation processing part 12, a pre-processing part 14, a reconstruction processing part 16, a color map displaying part 18, and an artificial intelligence 20. The controlling part 11 controls the CT imaging diagnostic aid system 10 to operate based on a user input. Under control of the controlling part 11, a non-contrast CT image is acquired.

The exponentiation processing part 12 is an example of a processing part which operates non-linear transformation (non-linear transformation processing part), and operates a processing of applying a non-linear transformation, for example, raising CT values of the acquired non-contrast CT image to a power. The pre-processing part 14 is performed prior to exponentiation processing. The pre-processing is performed for the purpose of preventing a CT image raised to a power from misleading the user or for the purpose of preventing a CT image raised to a power from being displayed unnaturally or abnormally. Specifically, it is possible to perform pre-processing of replacing CT value smaller than or equal to 0 with a value of zero or a near-zero value, pre-processing of replacing a CT value which causes overflow as a result of exponentiation with a value of 0 which does not cause overflow, pre-processing of shifting all CT values to a range in which an overflow does not occur, or the like. The reconstruction processing part 16 reconstructs the CT image based on the CT values raised to a power. The color map displaying part 18 displays the CT image which was reconstructed as a color image by comparing to color map.

The artificial intelligence 20 includes a model 22, a predicting part 24, and a learning processing part 26. The model 22 may, for example, be a neural network, whereas this is not a limitation. The model 22 is trained by the learning processing part 26. The learning processing part 26 uses training data and the corresponding teaching data to learn for parameters of the model 22. With the CT image which was reconstructed as training data and disease classification based on the CT image as teaching data, the model 22 enables prediction of disease classification from the CT image which was reconstructed. The prediction part 24 predicts disease classification based on the CT image which was reconstructed by inputting the CT image to the learned model 22. Examples of disease classification to be predicted include hyperacute cerebral infarction, subarachnoid hemorrhage, brain tumor, brain metastasis, liver metastasis, patient with arterial dissection, and the like.

The non-contrast CT imaging diagnostic aid system (method) of the present invention is particularly useful for diagnostic aid of diseases relying on a minute difference in CT values. For example, it is useful for non-contrast CT imaging diagnosis of a patient selected from the group consisting of non-contrast CT imaging diagnosis of hyperacute cerebral infarction, non-contrast CT imaging diagnosis of identification of a source of bleeding in subarachnoid hemorrhage, non-contrast CT imaging diagnosis of a spreading range of brain tumor, non-contrast CT imaging diagnosis of brain metastasis, non-contrast CT imaging diagnosis of liver metastasis, and non-contrast CT imaging diagnosis of detailed examination of a vascular wall of a patient with arterial dissection. Furthermore, it is useful for non-contrast CT imaging diagnosis of a patient selected from the group consisting of non-contrast CT imaging diagnosis of hyperacute cerebral infarction and non-contrast CT imaging diagnosis of brain tumor.

### Example

The present invention will now be described with reference to examples, whereas the present invention is not limited to the examples.

### Example 1

Each item of pixel data of a CT image is read. When a 16-bit integer overflow will occur as a result of raising CT values of a site to be analyzed to a power, the same numerical value is added to or subtracted from all the pixels, and thereafter the resultant value is raised to a power.

When the read pixel data type is a signed 16-bit integer, pixel values each having a value greater than or equal to 182 in terms of the CT value is replaced with 182, and when the read pixel data type is an unsigned 16-bit integer, pixel values each having a value greater than or equal to 256 is replaced with 256.

Furthermore, a pixel value smaller than or equal to 0 in terms of the CT value is replaced with 0.

To display the image of the pixel data raised to a power, a window level and a window width are set for display as in a typical CT. The window level is set to indicate the center position of the integer in which the image is displayed, and the window width is set to display a range centered on the window level, extending to the upper and lower halves of the window width.

For example, in a case in which the window level is 100 and the window width is 80, ranges from 60 to 140 in terms of the CT value are displayed. An image with a CT value at or below a lower limit is displayed as the lowest density, and an image with a CT value at or above an upper limit is displayed as the highest density.

When it is difficult to make a diagnosis with a grayscale, further improvement of diagnosability can be expected with a color map display.

When the displayed image is grainy, it is effective to use a smoothing filter.

### (1) Acquisition of non-contrast CT image

A CT image taken from a patient with suspected hyperacute cerebral infarction due to symptoms such as dysarthria, movement disorder, and disturbance of consciousness was used.

Head CT taken by using a CT scanner such as Aquilion ONE from Canon, Aquilion Lightning from Canon, or Definishon Flash from Siemens was used.

### (2) Raising CT value of each pixel in a CT image to a power

The matrix to the left of Figure 1 shows pixel values of the CT image. The matrix to the right is a matrix of the pixel data to the left raised to a power.

For example, raising data of 30 to the left to a power (to the power of two in this case) produces data of 900. Similarly, raising data of 35 to a power produces the value of 1225. The difference in signal values was 5 before raising to a power, whereas the difference in signal values increases to 325 through raising to a power.

For example, in a case in which the signal value of a normal tissue is 35 and the signal value of a cerebral infarction lesion is 30, only a difference of 5 integers at maximum can be displayed in the case of data before raising to a power, whereas it is possible to display a difference of 325 integers at maximum in the data after raising to a power.

### (3) Reconstruction of CT image based on resultant CT value raised to a power

Figure 2 shows the data in Figure 1 as CT images. The upper-left image is a diffusion-weighted image of MRI. The white portion in the right frontal lobe is a lesion site of acute-stage cerebral infarction.

Even as compared with the left brain, such difference is not perceptible in the CT image. However, in the image at the center, which is an image raised to a power, it is possible to clearly observe reduction in signal value of the right brain cortex. The obscuration of the lentiform nucleus is also clear as compared with the left brain.

The rightmost image is the image raised to a power and displayed in a color map.

The color map refers to what is made by preparing a color palette in which the ratio is varied from one specific color to another specific color and assigning a color to a value.

The early CT signs are clearly observable in both the image raised to a power and the image raised to a power in a color map display.

| | | |
|---|---|---|
| CT image | window level 40 | window width 120 |
| image raised to power | window level 1200 | window width 600 |
| image raised to power in color map | window level 1200 | window width 600 |

### Reference Signs List

- 10: non-contrast CT imaging diagnostic aid system
- 11: controlling part
- 12: exponentiation processing part
- 14: pre-processing part
- 16: reconstruction processing part
- 18: color map displaying part
- 20: artificial intelligence
- 22: model
- 24: predicting part
- 26: learning processing part

## Claims

1. A non-contrast CT imaging diagnostic aid system comprising: (1) means for acquiring a non-contrast CT image; and (2) means of applying a non-linear transformation to a CT value of each pixel in the CT image to reconstruct the CT image.

2. The non-contrast imaging diagnostic aid system according to claim 1, wherein the means of applying a non-linear transformation is means of raising a CT value of each pixel in a CT image to a power to reconstruct the CT image.

3. The non-contrast CT imaging diagnostic aid system according to claim 1 or 2, further comprising means of displaying the CT image which was reconstructed in a color map.

4. The non-contrast CT imaging diagnostic aid system according to any one of claims 1 to 3, wherein when applying a non-linear transformation to the CT value, at least one of pre-processing for replacing a CT value smaller than or equal to 0 with a value of zero or a near-zero value, pre-processing for replacing a CT value which causes overflow as a result of non-linear transformation with a value which does not cause overflow, and pre-processing for evenly shifting all CT values to a range in which an overflow does not occur is performed.

5. The non-contrast CT imaging diagnostic aid system according to any one of claims 1 to 4, wherein the non-contrast CT image is a non-contrast CT image of a patient selected from the group consisting of a non-contrast CT image of hyperacute cerebral infarction, a non-contrast CT image of subarachnoid hemorrhage, a non-contrast CT image of brain tumor, a non-contrast CT image of brain metastasis, a non-contrast CT image of liver metastasis, and a non-contrast CT image of detailed examination of a vascular wall of a patient with arterial dissection.

6. The non-contrast CT imaging diagnostic aid system according to any one of claims 1 to 4, wherein the non-contrast CT image is a non-contrast CT image of a patient selected from the group consisting of a non-contrast CT image of hyperacute cerebral infarction and a non-contrast CT image of brain tumor.

7. The non-contrast CT imaging diagnostic aid system according to any one of claims 1 to 6, further comprising: an artificial intelligence built through learning by using disease classification based on a non-contrast CT image as teaching data and using a CT image reconstructed by the means of reconstructing as training data; and means of predicting disease classification based on the non-contrast CT image acquired by the means for acquiring by reconstructing the non-contrast CT image by the means of reconstructing, and inputting the reconstructed non-contrast CT image to the artificial intelligence.

8. A non-contrast CT imaging diagnostic aid method, comprising: (1a) a step of acquiring a non-contrast CT image; and (2a) a step of applying a non-linear transformation to a CT value of each pixel in the CT image to reconstruct the CT image.

9. The non-contrast imaging diagnostic aid method according to claim 8, wherein the step of applying a non-linear transformation is a step of raising a CT value of each pixel in the CT image to a power to reconstruct the CT image.

10. The non-contrast CT imaging diagnostic aid method according to claim 8 or 9, further comprising means of displaying the CT image which was reconstructed in a color map.

11. The non-contrast CT imaging diagnostic aid method according to any one of claims 8 to 10, wherein when applying a non-linear transformation to the CT value, at least one of pre-processing for replacing a CT value smaller than or equal to 0 with a value of zero or a near-zero value, pre-processing for replacing a CT value which causes overflow as a result of non-linear transformation with a value which does not cause overflow, and pre-processing for evenly shifting all CT values to a range in which an overflow does not occur is performed.

12. The non-contrast CT imaging diagnostic aid method according to any one of claims 8 to 11, wherein the non-contrast CT image is a non-contrast CT image of a patient selected from the group consisting of a non-contrast CT image of hyperacute cerebral infarction, a non-contrast CT image of subarachnoid hemorrhage, a non-contrast CT image of brain tumor, a non-contrast CT image of brain metastasis, a non-contrast CT image of liver metastasis, and a non-contrast CT image of detailed examination of a vascular wall of a patient with arterial dissection.

13. The non-contrast CT imaging diagnostic aid system according to any one of claims 8 to 11, wherein the non-contrast CT image is a non-contrast CT image of a patient selected from the group consisting of a non-contrast CT image of hyperacute cerebral infarction and a non-contrast CT image of brain tumor.

14. The non-contrast CT imaging diagnostic aid method according to any one of claims 8 to 13, further comprising: an artificial intelligence built through learning by using disease classification based on the non-contrast CT image as teaching data and using a CT image reconstructed by the step of reconstructing as training data; and a step of predicting disease classification based on the non-contrast CT image acquired by the step of acquiring by reconstructing the non-contrast CT image by the step of reconstructing, and inputting the reconstructed non-contrast CT image to the artificial intelligence.
